# EUROPEAN PATENT APPLICATION

(11) **EP 4 772 882 A1**
(43) Date of publication of application: **08.07.2026**
(21) Application number: 25150038.5
(22) Date of filing: 02.01.2025
(51) Int. Cl.: G01N 33/543, G01N 33/58

(54) **TOTAL EXOSOME DETECTION ON AUTOMATED ROUTINE IMMUNOASSAY ANALYZERS**

(71) Applicant: Siemens Healthineers AG, 91301 Forchheim (DE)
(72) Inventor: Brock, Jens-Peter, 90579 Langenzenn (DE); Huang, Yiwei, 91058 Erlangen (DE); Hurler, Lisa, 90459 Nürnberg (DE); Dethlefsen, Mark, 91058 Erlangen (DE)
(74) Representative: Siemens Healthineers Patent Attorneys

(57) **Abstract**

The present invention relates to a method for detecting exosomes in a sample comprising providing the exosome in an aqueous solution; interacting the exosome with at least one first interactor molecule, wherein the interaction yields a complex comprising both the exosome and the first interactor molecule; interacting the complex of the previous step with at least a second interactor molecule linked to a chemiluminescent dye; interacting the complex of the previous step with a substrate or a bead and thereby immobilizing it; performing a washing step; identifying the complex of the previous step by performing a chemiluminescent reaction, wherein the identification results in a chemiluminescent emission of photons; and detecting the emission of photons. Also envisaged is a corresponding kit for detecting exosomes in a sample.

## Description

### TECHNICAL FIELD

The present invention relates to a method for detecting exosomes in a sample comprising providing the exosome in an aqueous solution; interacting the exosome with at least one first interactor molecule, wherein the interaction yields a complex comprising both the exosome and the first interactor molecule; interacting the complex of the previous step with at least a second interactor molecule linked to a chemiluminescent dye; interacting the complex of the previous step with a substrate or a bead and thereby immobilizing it; performing a washing step; identifying the complex of the previous step by performing a chemiluminescent reaction, wherein the identification results in a chemiluminescent emission of photons; and detecting the emission of photons. Also envisaged is a corresponding kit for detecting exosomes in a sample.

### BACKGROUND

Exosomes are the smallest class of cell-derived extracellular vesicles (EVs) with endosomal origin and are typically 30-150 nm in diameter. Exosomes have typically a lipid bilayer membrane and comprise a complex cargo of contents derived from the original cell, e.g. mRNA, miRNA, DNA and proteins. On the exosomal surface (trans-) membrane proteins are detectable.

Exosomes can be found in almost any body fluid such as cerebrospinal fluid (CSF), blood, urine, sperm and tear fluid and have similarities with their origin cell. Exosomes derived from different tissues will thus show different compositions. For example, an exosome originating from a liver cell may have a different composition than a brain derived exosome.

Due to the association with several diseases, exosomes are believed to have the potential to break through various diagnostic barriers, such as early detection of cancer and neurodegenerative diseases. Some diseases lead to an increase or decrease in the total number of exosomes in the bloodstream; others lead to an abnormal composition of the exosome cargo.

However, it is still difficult to isolate exosomes reliably, time-efficiently and cost-effectively. Many different isolation techniques are commercially available. One example is the size exclusion chromatography technology as developed and commercialized by IZON Science. An example for quantification is the ExoTEST for Overall Exosomes capture and quantification from Hansabiomed. None of the currently available exosome isolation and quantification methods techniques are, however, currently capable of fully exploiting the diagnostic potential of exosomes.

Furthermore, the assessment of the quality and quantity of isolated exosomes is indispensable but difficult due to the small size of the vesicles, their heterogeneity, and the usually low quantities of analyte. Moreover, missing automation and standardization between different laboratories lead to major difficulties in terms of comparability.

There is hence a need for a reliable, sensitive, and easily automatable method for detecting exosomes.

### SUMMARY

The present invention addresses this need and provides in a first aspect a method for detecting exosomes in a sample comprising the steps: (i) providing the exosome in an aqueous solution; (ii) interacting the exosome with at least one first interactor molecule, wherein the interaction yields a complex comprising both the exosome and the first interactor molecule; (iii) interacting the complex of step (ii) with at least a second interactor molecule linked to a chemiluminescent dye; (iv) interacting the complex of step (ii) or (iii) with a substrate or a bead and thereby immobilizing it; (v) performing a washing step; (v) identifying the complex of step (iv) by performing a chemiluminescent reaction, wherein the identification results in a chemiluminescent emission of photons; and (vii) detecting the emission of photons. This method advantageously allows to measure exosomes without the need for pre-analytical steps such as column-based purification or ultracentrifugation which are usually employed in commercially available assays. It further is capable of quantifying pre-isolated exosomes. Since the method is automatable, the quantification can be performed in a certified, highly stable and reliable system and a standardization of exosome quantification can be achieved between different labs and different countries.

In a preferred embodiment the immobilization of the complex of step (ii) or (iii) is performed with a magnetic bead via a third interactor molecule which is present at the substrate or bead; or via the first interactor which is immobilized at the substrate ab initio.

In a further preferred embodiment, the first interactor molecule comprises biotin and the third interactor molecule comprises streptavidin and the interaction is a biotin-streptavidin binding.

In yet another preferred embodiment the immobilization is performed via a magnetic force.

In a further preferred embodiment steps (ii) and (iii) of the method are performed simultaneously, sequentially ((ii) before (iii)) or in reverse order ((iii) before (ii)), optionally followed and/or interrupted by one or more washing and/or incubation step(s).

In a further preferred embodiment the first and/or second interactor molecule is a binding protein. It is particularly preferred that the binding protein is an antibody.

In a more preferred embodiment the antibody is an antibody against a specific antigen present at the surface of an exosome, such as a tetraspanin.

According to the present invention, it is further preferred that the first and/or second interactor molecules are binding proteins or antibodies against at least three different antigens present at the surface of an exosome, preferably three different tetraspanins.

In particularly preferred embodiments the antibody is an anti-CD81 antibody, an anti-CD63 antibody or an anti-CD9 antibody.

In further preferred embodiments the chemiluminescent dye is an acridinium ester, preferably NSP-DMAE, HQYAE, TSPAE, NSP-LEZAE, Rhodamine-2-AM-DMAE or CNF-2-AM-DMAE.

In yet another preferred embodiment the acridinium ester is covalently linked to the binding protein. The binding protein is, in a particularly preferred embodiment, an antibody.

In further preferred embodiments the detection is a quantitative detection or a semi-quantitative detection.

It is further preferred that the sample is a plasma, serum, urine, saliva, or cell culture media, or a fluid sample which comprises exosomes. Such a fluid sample is preferably a biofluid sample.

In a further aspect the present invention relates to a kit for detecting exosomes in a sample comprising (1) a calibrator solution comprising a predefined number of exosomes; (2) a first interactor molecule, which is capable of interacting with an antigen at the surface of an exosome and the first interactor molecule; (3) a second interactor molecule, which is capable of interacting with an antigen at the surface of an exosome and which is linked to a chemiluminescent dye; (4) a magnetic bead comprising a third interactor molecule which is capable of interacting with the first interactor molecule by a biotin-streptavidin binding, wherein the first and second interactor molecule is an antibody against an exosome surface marker, preferably against tetraspanin.

### BRIEF DESCRIPTION OF THE DRAWINGS

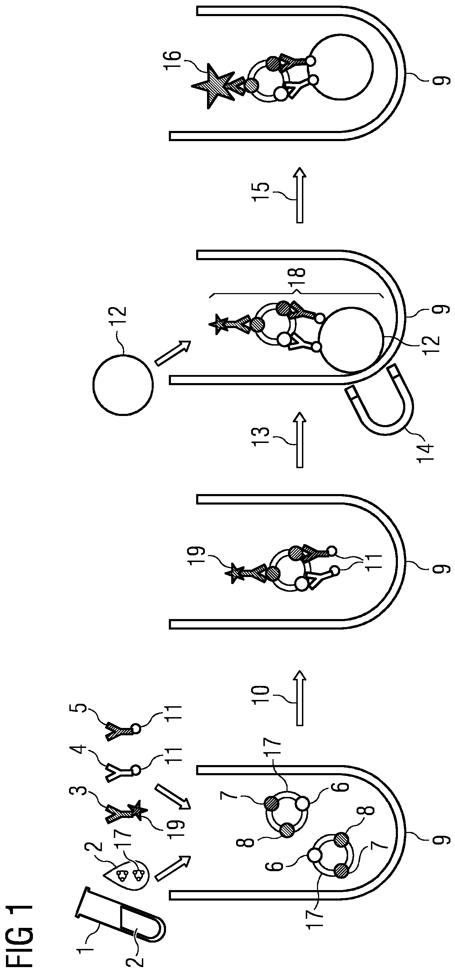

FIG. 1 shows an overview of the steps of one embodiment of the present invention. Depicted is method which starts with a sample in vial (1), which comprises exosomes (17) and is filled (2) into a reaction vessel (9). The exosome contains different surface markers (tetraspanins) (6, 7, 8). The sample is contacted with first interactor molecules (4 and 5) as well as second interactor molecules (3). The mixture is incubated (10) which leads to a binding of first and second interactor molecules to the different surface markers (tetraspanins) on the exosomes. The first interactor molecules (4 and 5) comprise additionally a biotin (11) and the second interactor molecules (3) comprise additionally a chemiluminescent dye (19). Upon further incubation and the addition of a magnetic bead (12) a complex (18) is formed. The complex is immobilized via magnetic force (14) and subsequently washed (15). In a last step a chemiluminescent reaction (16) is performed which produces light, that can be detected.
FIG. 2 shows exosome signals observed in an Atellica assay according to the present invention when measuring plasma (Plasma Pool 1 (P1) (21)), ExoStandard (22), and BSA in PBS (1% BSA in PBS; background signal; 23). The ordinate shows RLU units (20).
FIG. 3 shows exosome precipitation from human plasma. Exosomes were precipitated from 1 mL human plasma (input (24)) using the ThermoFisher Total Exosome Isolation Kit (from plasma (26)) according to manufacturer's instructions. Further depicted are output (25) comprising supernatant (exosome free) (28) and precipitate (exosomes) (27), as well as BSA in PBS (1% BSA in PBS; background signal; 29). The ordinate shows RLU units (20).
FIG. 4 depicts a serial dilution measurement of ExoStandard in PBS. Shown is the linearity of ExoStandard (ExoStd) serially diluted in PBS. The ordinate shows RLU units (20) and abscissa the dilution (30).
FIG. 5 depicts a serial dilution measurement of plasma in PBS. Shown is the linearity of plasma serially diluted in PBS. The ordinate shows RLU units (20) and abscissa the dilution (30).
FIG. 6 shows the measurement of ExoStandard serial dilution at an Atellica apparatus. The ordinate shows RLU units (20) and the abscissa the total weight of the exosomes in µg (31). The curve has the following parameters: y=12709x + 1294.4 and R² = 0.9988.
FIG. 7 shows the measurement of the same ExoStandard serial dilution as in FIG. 6 in an ELISA assay. The ordinate shows RLU units (20) and the abscissa the total weight of the exosomes in µg (31). The curve has the following parameters: y=0,00038x + 0.015 and R² = 0.8716.
FIG. 8 shows the results of an intra-assay variation test. Depicted are measurement 1 (34), measurement 2 (35), measurement 3 (36), measurement 4 (37), measurement 5 (38), measurement 6 (39) of 1% BSA in PBS (32), P1 IZON (33) and P1 Plasma (34). The ordinate shows RLU units (20).

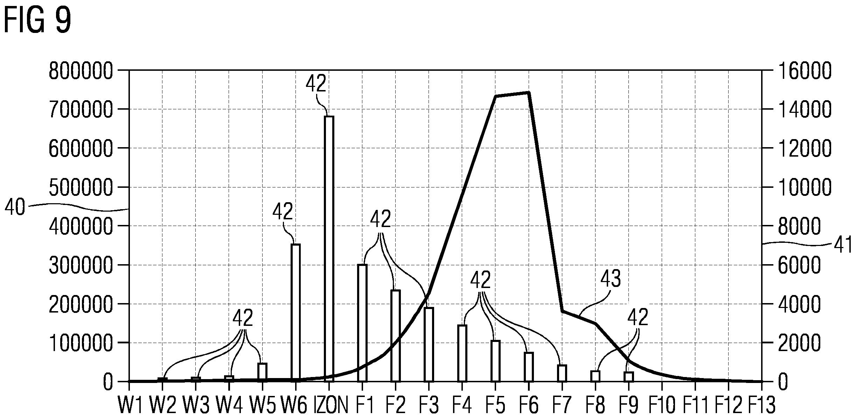

FIG. 9 shows the results of an IZON fractionation comprising exosomes and proteins in IZON fractions. Depicted on the abscissa are the fractions comprising exosomes (42) and protein (43). The left ordinate shows exosomes (RLU x volume) (40) and the right ordinate shows protein (µg) (41). Exosomes were isolated using size exclusion chromatography following instructions by the manufacturer (IZON). Wash fractions (W1-W6), the IZON fraction (IZON), as well as post-IZON fractions (F1-F13) were collected and analyzed using the assay according to the present invention based on an Atellica device. The result is provided as RLUs for exosomes (42) and BCA for total protein content (43).
FIG. 10 shows the results of an IZON fractionation comprising exosomes in IZON fractions. Depicted are the input (24), which is plasma (44) and the output (25) comprising several fractions. The ordinate shows exosomes (RLU x volume) (40). Exosomes were isolated using size exclusion chromatography following instructions by the manufacturer (IZON). Wash fractions (W1-W6), the IZON fraction (IZON), as well as post-IZON fractions (F1-F13) were collected and analyzed using the assay according to the present invention based on an Atellica device.

### DETAILED DESCRIPTION OF EMBODIMENTS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" or "essentially consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "(i)", "(ii)", "(iii)" or "(a)", "(b)", "(c)", "(d)", or "first", "second", "third" etc. and the like in the description or in the claims, are used for distinguishing between similar or structural elements and not necessarily for describing a sequential or chronological order.

It is to be understood that the terms so used are interchangeable under appropriate circumstances and that the embodiments of the invention described herein are capable of operation in other sequences than described or illustrated herein. In case the terms relate to steps of a method, procedure or use there is no time or time interval coherence between the steps, i.e., the steps may be carried out simultaneously or there may be time intervals of seconds, minutes, hours, days, weeks etc. between such steps, unless otherwise indicated.

It is to be understood that this invention is not limited to the particular methodology, protocols etc. described herein as these may vary. It is also to be understood that the terminology used herein is for the purpose of describing particular embodiments only and is not intended to limit the scope of the present invention that will be limited only by the appended claims.

The drawings are to be regarded as being schematic representations and elements illustrated in the drawings are not necessarily shown to scale. Rather, the various elements are represented such that their function and general purpose become apparent to a person skilled in the art.

Unless defined otherwise, all technical and scientific terms used herein have the same meanings as commonly understood by one of ordinary skill in the art.

Independent of the grammatical term usage, individuals with male, female or other gender identities are included within the term.

As has been set out above, the present invention concerns in one aspect a method for detecting exosomes in a sample comprising the steps: (i) providing the exosome in an aqueous solution; (ii) interacting the exosome with at least one first interactor molecule, wherein the interaction yields a complex comprising both the exosome and the first interactor molecule; (iii) interacting the complex of step (ii) with at least a second interactor molecule linked to a chemiluminescent dye; (iv) interacting the complex of step (ii) or (iii) with a substrate or a bead and thereby immobilizing it; (v) performing a washing step; (vi) identifying the complex of step (iv) by performing a chemiluminescent reaction, wherein the identification results in a chemiluminescent emission of photons; and (vii) detecting the emission of photons.

The term "exosome" as used herein relates to an extracellular vesicle means a vesicle originating as ESCRT-dependent invaginations of early endosomes that are released into circulation upon fusing of the resultant multi-vesicular bodies (MVBs) with the plasma membrane. In certain embodiments, the term also refers to similar vesicles, which are known as ectosomes, exomeres or oncosomes (see, for example, Rojalin et al., 2019, Front. Chem., 7, 279 for further details). In further embodiments, an exosome as defined in the present invention may have the format and properties of a microvesicle. The size of an exosome according to the present invention typically ranges from about 30 to about 150 nm. An exosome as understood within the context of the present invention may be differentiated from extracellular vesicles (EVs) which may have a larger size, e.g. up to 500 nm, 750 nm or 1000 nm. Exosomes are typically generated in the endosomal compartment of cells by invagination of the cell membrane (endocytosis) and the subsequent formation of intracellular vesicles, which are released toward the extracellular space in response to different signals (exocytosis). The signals for exosome release may be stressful cellular conditions (e.g. hypoxic situations), pH or ionic alterations, viral infection, ischemia, phosphatidylinositol 3-kinase and loose cell-to-cell adhesion. As a result of the cell membrane budding, the molecular composition varies depending on the origin cell. As such, exosomes are typically surrounded by a lipid bilayer with characteristic and cell-specific membrane proteins, such as tetraspanins, integrins, various intercellular adhesion molecules or the major histocompatibility complex. Moreover, they contain a variety of molecules, such as lipids, proteins and nucleic acids, including messenger-RNA (mRNA) and microRNA (miRNA). The identity and amount of these molecules may vary depending on the origin and formation process of the exosome. The specific biochemical and molecular composition of exosomes may thus serve as diagnostic marker and may accordingly be used for diagnostic or differential detection approaches. In certain embodiments, the present invention further envisages a size separation of exosomes as defined herein above from EVs having a different, e.g. larger size of e.g. 500 to 1000 nm. To achieve such a size separation the method according to the present invention may comprise an optional further, preferably initial, step in which exosomes are separated from other EVs according to their size. Preferred is the performance of Size Exclusion Chromatography. A preferred example of such an approach is the Size Exclusion Chromatography technique offered by IZON Science. Also envisaged is any alternative technique known to the skilled person.

The term "interactor molecule" as used herein refers to any chemical or biochemical entity which is capable of interacting with an exosome. The interaction is preferably a binding, in particular a specific binding. Preferred interactors are binding proteins such as antibodies, antibody fragments, lectins and the like, as well as small molecules, which are able to bind, for example, to proteins or peptides. Also envisaged are molecularly imprinted polymers (MIPs), i.e., "smart materials" with potential to replace antibodies in the future. The concept of the MIP technology essentially relies on the formation of a synthetic polymer around a template molecule, which after removal, leaves behind an idealized imprint; cavities within the polymer matrix, which can specifically recognize the template molecule. It is preferred that the interactor is an antibody.

In a first step the exosome is provided in an aqueous solution. The solution may, for example, comprise or be composed of suitable buffers such as a buffer suitable for immunologic interactions etc. The solution is typically suitable for the performance of interactions between an exosome and an interactor, e.g., for the binding of an antibody. Accordingly, salt concentration, pH and suitable buffer conditions etc. may preferably be adjusted to the envisaged interactions. In further embodiments additional conditions may be observed and suitably adjusted such as temperature of the reaction environment, light exposure, agitation or liquid flow velocity in the reaction mix and the like.

The exosome may be derived from a sample. The term "sample" as used herein relates to any fluid or biological material obtained via suitable methods known to the person skilled in the art from a subject. The sample used in the context of the present invention should preferably be collected in a clinically acceptable manner, more preferably in a way that exosomes are preserved. The sample may generally be any fluid, preferably biofluid, which comprises exosomes. For example, the samples may be a plasma, serum, or cell culture media sample. Also envisaged are body tissues and/or fluids, such as sweat, sputum or saliva, semen and urine, as well as feces or stool samples. The present invention envisages the use of non-pooled samples. In a specific embodiment of the present invention the content of a sample may be submitted to a specific pre-enrichment step. In further embodiments of the invention, biopsy or resections samples may be obtained and/or used.

The provision of the exosome in an aqueous solution may take place in any suitable environment, e.g., a reaction chamber, a reaction vessel, in a reaction zone in a device such as a microfluidic device or the like. It is preferred that the provision of the exosome takes place in a cuvette. The reaction vessel may, in certain embodiments, comprise a substrate which allows for the immobilization of certain ingredients or components, as defined herein.

In a subsequent step of the method an interaction of the exosome(s) with at least one first interactor molecule is initiated. This initiation takes place by introducing the exosome, e.g., in the form of a sample or derived from a sample, into an aqueous solution, or by introducing the interactor into an aqueous solution comprising the exosome(s). The interaction leads to the building of a complex between interactor(s) and exosome(s). For example, the complex may be a binding complex between an antibody as interactor molecule and its antigen as part of the exosome.

Preferably, the interaction is an immunologic binding reaction between an exosome and the interactor molecule being an antibody. The interaction yields a complex comprising both the exosome and the first interactor molecule.

For example, the first interactor molecule may be a binding protein such as an antibody, which is capable of specifically binding to the exosome, e.g. by binding to a specific protein, antigen or epitope present at the surface of the exosome which is exposed.

The first interactor molecule may have different forms, i.e. the term relates to at least one binding protein but also envisages several or a multitude of different binding proteins, e.g. antibodies. For example, one first interactor may be a binding protein for one specific protein, antigen or epitope on the surface of an exosome, a second first interactor may be a different binding protein for another specific protein, antigen or epitope on the surface of an exosome, etc.

In preferred embodiments, the first interactor molecule is a binding protein capable of specifically binding to a protein present at the surface of an exosome. Examples of such proteins include tetraspanins. The term "tetraspanin" as used herein refers to a membrane protein found in all multicellular eukaryotes, which belongs to a family of similar proteins (tetraspanin protein family or transmembrane 4 superfamily). The tetraspanins typically have four transmembrane alpha-helices and two extracellular domains, a small extracellular domain or loop, (SED/SEL or EC1) and a large extracellular domain/loop (LED/LEL or EC2). Tetraspanins display numerous properties including functions in cell adhesion, motility, activation, and proliferation. They are also assumed to be involved in pathological conditions such as metastasis or viral infection. Tetraspanins are further assumed to regulate the trafficking of selective associated proteins and may have a function in membrane compartmentalization. Several tetraspanins are major constituents of exosomes. Examples of these exosome associated tetraspanins include CD9, CD81, CD63, CD82, CD53, and CD37. Further information on the identity of tetraspanins in exosomes would be known to the skilled person or can be derived from suitable literature sources such as Jankovicova et al., 2020, International Journal of Molecular Science, 21, 7568; or Andreu and Yanez-Mo, 2014, Front. Immunol., 5, 442.

In preferred embodiments of the present invention the first interactor molecule is a binding protein, e.g. antibody, which is capable of specifically binding to any tetraspanin present on an exosome. According to specific embodiments of the invention the binding proteins or antibodies are directed against at least two different antigens present at the surface of an exosome. The antigens are represented by two or more different tetraspanins.

Particularly preferred are first interactor molecules, e.g. antibodies, being capable of specifically binding to the tetraspanin CD9, CD81 or CD63.

In further particularly preferred embodiments, the first interactor molecule is a group of binding proteins, e.g. antibodies, wherein one group member binds to CD9, another group member binds to CD81 and yet another group member binds to CD63. Further envisaged are groups with binding proteins only for two or one of the three tetraspanins CD9, CD81 or CD63. Also envisaged are groups comprising binding proteins for one, two or more additional tetraspanin proteins.

In further particularly preferred embodiments the first interactor molecule is a binding protein, e.g. antibody, against CD63 or CD9. Also preferred is the employment of the group of first interactor molecules comprising a binding protein, e.g. antibody, against CD63 and a binding protein, e.g. antibody, against CD9. It is further preferred that at least two different tetraspanins are bound and recognized by interactor molecules according to the present invention.

In a further step of the method the complex of the previous step as mentioned above is identified with a second interactor linked to a chemiluminescent dye. The term "second interactor" as used herein refers to a chemical or biochemical entity, which is capable of a specific interaction with the complex of the first interactor and the exosome(s). This specific interaction is preferably a specific binding, which requires a certain degree of complex formation, or the exposure of a certain site or epitope on the exosome.

For example, the second interactor may be a binding protein such as an antibody, which is capable of specifically binding to a complex between the first interactor and the exosome, or to a specific protein, antigen or epitope present on or at the surface of the exosome.

It is particularly preferred that the second interactor is an antibody which is capable of specifically binding to a tetraspanin being present at the surface of an exosome, as described above. Particularly preferred are tetraspanins CD9, CD63 and CD81. Accordingly, second interactor molecules, e.g. antibodies, are preferred which are capable of specifically binding to the tetraspanin CD9, CD81 or CD63.

In the most preferred embodiment, the second interactor is a binding protein, e.g. antibody, capable of binding to tetraspanin CD81.

Also envisaged is the employment of a group of second interactor molecules, wherein one group member binds to one tetraspanin, e.g. CD9, another group member binds to another tetraspanin, e.g. CD81 and yet another group member binds to a third tetraspanin, e.g. CD63, etc. Further envisaged are smaller or larger groups comprising second interactor molecules capable of binding to a subset of the mentioned tetraspanins or one or more additional tetraspanins as described herein or known to the skilled person. It is preferred that members of groups of second interactor molecules being capable of binding to different tetraspanins comprise different chemiluminescent dyes, e.g. different acridinium ester dyes.

It is particularly preferred that the first or second interactor molecule is an anti-CD81 antibody, an anti-CD63 antibody or an anti-CD9 antibody.

The term "chemiluminescence" as used herein refers to the emission of light, i.e., luminescence, as the result of a chemical reaction. In general, a chemiluminescent reaction can be generated in a direct reaction or indirect reaction, wherein, two reagents, usually a reagent or chemiluminescent dye and an oxidant in the presence of cofactors, react to from a product or intermediate, optionally in the presence of a catalyst. A fraction of the product or intermediate will be formed in an electronically excited state, which can subsequently relax to the ground state with emission of a photon. The reagent is typically a precursor, which is converted into an electronically excited molecule, responsible for light emission. A catalyst enzyme or metal ions may reduce the activation energy and provide an adequate environment for producing high chemiluminescence efficiency out of the process. In certain embodiments cofactors may be used to convert one or more of the reagents into a form capable of reacting and interacting with the catalyst, or to provide an efficient leaving group if bond cleavage is required to produce the excited emitter. Chemiluminescence in aqueous environment is typically caused by redox reactions.

The term "chemiluminescent dye" as used herein refers to an agent which, upon a chemical reaction, emits electromagnetic radiation in the ultraviolet or visible light range. Examples of chemiluminescent dyes include luminol, imidazole, or acridinium esters. Particularly preferred is the use of acridinium esters. Acridinium ester or AE are currently the most commonly used direct chemiluminescent dyes since they advantageously show high stability, low background, high signal-noise ratio, quick light-emitting and a high luminous efficiency. Typically, AE independently emits light in the presence of H₂O₂ and NaOH without the participation of an enzyme and reaches its maximum chemiluminescence in about 0.4 seconds. Thus, exposing the AE to an alkaline H₂O₂ solution typically triggers a flash. Before the flash occurs, the acridone, which is the light emitting species, separates from the remainder of the conjugate, so the subsequent light emission is not influenced by further components. It is preferred that the trigger solution comprises a detergent, which improves the reaction speed. Particularly preferred is the detergent ARQUAD 16-50.

Preferred is the use of chemiluminescent dyes which emit light in different wavelengths, preferably in the range of 400 to 430 nm, e.g., at 425 nm; 450 to 500 nm, e.g., 475 nm, 500 to 550 nm, e.g., 525 nm; 600 to 650 nm, e.g., 625 nm or 675 to 725 nm, e.g., 700 nm. Preferred is the employment of acridinium ester chemiluminescent dyes such as DMAE, NSP-DMAE, HEGAE, HQYAE, ZAE, TSPAE, NSP-LEZAE, Rhodamine-2-AM-DMAE or CNF-2-AM-DMAE, or analogues thereof. Particularly preferred is the use of NSP-DMAE, HQYAE, TSPAE, NSP-LEZAE, Rhodamine-2-AM-DMAE or CNF-2-AM-DMAE. NSP-DMAE is a chemiluminescent dye which emits light with a peak wavelength of 427 nm. TSPAE is a chemiluminescent dye which emits light with a peak wavelength of 480 nm. HQYAE is a chemiluminescent dye which emits light with a peak wavelength of 480 nm. NSP-LEZAE is a chemiluminescent dye which emits light with a peak wavelength of 522 nm. Rhodamine-2-AM-DMAE is a chemiluminescent dye which emits light with a peak wavelength of 620 nm. CNF-2-AM-DMAE is a chemiluminescent dye which emits light with a peak wavelength of 718 nm. Also envisaged are analogues of said AEs or other chemiluminescent dyes, including future developments of these or similar dyes.

The chemiluminescent dye, e.g. acridinium ester, as described above is preferably linked to the second interactor molecule via a covalent bond.

In a further step (iv), the complex of step (ii) or (iii) is interacted with a substrate or a bead and thereby it is immobilized. The interaction is preferably performed via a third interactor molecule. The third interactor molecule comprises an element, which is capable of binding to biotin.

The present invention envisages in corresponding embodiments that the first interactor molecule is biotinylated or comprises biotin, which allows the third interactor to bind to the first interactor molecule. Biotin is a heterocyclic compound, with a sulfur-containing tetrahydrothiophene ring fused to a ureido group, wherein a C5-carboxylic acid side chain is appended to the ring. It is typically used a biotinylation of further molecule, e.g. a protein, i.e. the biotin is covalently attached to said further molecule. The biotinylation typically used for subsequent interaction with an avidin or similar molecule, in particular, streptavidin wherein biotin binds to avidin and similar molecules with an extremely high affinity, fast on-rate, and high specificity.

In preferred embodiments, the element which is capable of binding to the biotin is an avidin or a molecule similar to avidin. Avidin is a tetrameric glycoprotein composed of monomers consisting of 128 amino acids. An avidin variant preferably envisaged by the present invention is streptavidin, which is produced by Streptomyces bacteria. Another avidin variant that can be used in the context of the present invention is tamavidin, e.g. tamavidin 1 or tamavidin 2. Further, avidin variant shwanavidin may be used. Also envisaged are avidin variants rhizavidin, bradavidin, burkavidin, zebavidin, xenavidin or strongavidin. It is preferred that streptavidin is used for the interaction with biotin on the first interactor molecule.

The term "substrate" as used herein refers to a solid phase present at the surface of an entity, which is typically composed of porous and/or non-porous material, usually insoluble in water. The substrate may have various forms such as a vessel, tube, microtitration plate, or cartridge etc. Usually, the surface of the solid phase is hydrophilic. The substrate may be composed of various materials such as inorganic materials and/or organic materials, synthetic materials, naturally occurring materials and/or modified naturally occurring materials. Examples of substrate materials include polymers such as cellulose, nitrocellulose, cellulose acetate, polyvinyl chloride, polyacrylamide, crosslinked dextran molecules, agarose, polystyrene, polyethylene, polypropylene, polymethacrylate, or nylon; ceramics; silicate; glass; metals, e.g., noble metals such as gold and silver; or mixtures or combinations thereof. It is preferred that the substrate is localized at the inner surface of the vessel, preferably cuvette as described herein.

The term "bead" is used herein refers to a spherical entity which typically has a size of 0.1-150 µm. The bead may, for example, have a high binding capacity due to being porous, creating an enlarged surface area. In particularly preferred embodiments, the bead is a magnetic bead.

The term "magnetic bead" refers to a magnetic particle. It may be composed of or comprise iron oxide such as Fe₃O₄, or Fe₂O₃, or iron platinum. Also envisaged are alloys with Ni, Co and Cu, or particles comprising these elements. In certain embodiments, the magnetic bead may comprise a certain amount of superparamagnetic material, e.g., 10%, 20%, 30%, 40%, 50%, 60%, 70%, 80%, 90% or more than 90% by weight. The beads may, for example, comprise an encapsulation with a polymer coating such as polystyrene. In preferred embodiments, the material comprised in the magnetic particle may have a saturated moment per volume as high as possible thus allowing to maximize gradient related forces.

The magnetic beads may have any suitable form, e.g., be of a symmetrical, globular, essentially globular or spherical shape, or be of an irregular, asymmetric shape or form. The size of a magnetic bead envisaged by the present invention may range between 50 nm and 1500 nm. Preferred are magnetic beads in the nanometer range. In preferred embodiments the magnetic bead diameter is larger than 100 nm. Particularly preferred are magnetic beads of a size of 300 nm to 1000 nm, e.g., 300 nm, 400 nm, 500 nm, 600 nm, 700 nm, 800 nm, 900 nm, or 1000 nm, or any value in between.

Accordingly, substrate or bead as defined above may be connected, e.g. via covalent binding, to a third interactor molecule, which is preferably streptavidin. The presence of the third interactor molecule, e.g. streptavidin, allows for an interaction of the third interactor molecule with the biotin present at the first interactor molecule. In case of the third interactor molecule being present at the substrate, the biotin-third interactor molecule interaction leads to an immobilization of the complex comprising the first interactor molecule and the exosome or comprising the first and second interactor molecule and the exosome to the substrate.

In an embodiment in which the third interactor molecule is present at a magnetic bead, the biotin-third interactor molecule interaction leads to a binding of the complex comprising the first interactor molecule and the exosome or comprising the first and second interactor molecule and the exosome to the magnetic bead. The magnetic bead may then be immobilized, e.g. to the vessel or cuvette, by magnetic force, i.e. magnetic beads - magnetic force interaction.

The present invention thus envisages a method in which the first interactor molecule, e.g., an antibody, is immobilized at a substrate, e.g., at the inner surface of the cuvette as described herein, or on a bead such as a magnetic bead. For example, the first interactor may be immobilized to said substrate, e.g., via a streptavidin-biotin linkage. The complex formation with the exosome, preferably by an interaction of the first interactor molecule, e.g. antibody, with a tetraspanin present at the surface of the exosome, may accordingly take place at the substrate or bead, e.g. magnetic bead, followed by an interaction with the second interactor, e.g., a further antibody which is also specific for the exosome, preferably binding to a different tetraspanin as described above, which is linked to a chemiluminescent dye.

In variant embodiments the first interactor and the third interactor may be connected ab initio, i.e., the first interactor may be bound via its binding unit, e.g., biotin, to the third interactor, e.g., streptavidin, prior to the interaction with the exosome.

In another variant embodiment the second interactor molecule, e.g., an antibody which is specific for the exosome, preferably binding to a tetraspanin as described above, which is linked to a chemiluminescent dye is interacted with the exosome. Subsequently, the first interactor molecule, e.g., an antibody, is added to the complex, wherein the first interactor molecule, e.g. antibody, is also interacting, i.e. binding to the exosome preferably via a different tetraspanin present at the surface of the exosome. The first interactor comprising biotin may further interact via the third interactor, e.g. streptavidin, with the substrate or a magnetic bead. An immobilization of the complex of the first and second interactor molecule and the exosome may thereby take place at the substrate, or alternatively, at the bead, preferably on the magnetic bead upon usage of magnetic force. The magnetic force accordingly immobilizes the complex to the vessel, e.g. the wall of the cuvette as described herein.

It is particularly preferred that the immobilization is performed by using magnetic force to attract and bind the magnetic bead as defined herein comprising a complex of an exosome and the interactor molecules as defined herein to a surface, e.g. a cuvette.

Subsequently, a washing step (v) is performed. This step allows to remove excess interactors or other components which are not part of the immobilized complex to be detected, e.g. second interactor molecules which are excessive and/or are not bound to the complex of the previous step or other components which are not part of the complex to be detected. The washing thereby reduces the likelihood of incorrect detection in the subsequent steps. The washing may be performed according to any suitable procedure.

It is preferred that the sequence of interactions is firstly the formation of the complex as defined herein, followed by an immobilization, e.g. via magnetic forces, or interaction at a surface, and subsequently a washing step. In certain specific embodiments, the immobilization of interactor molecules to a substrate may take place before the complex is formed or at the same time.

In a further step (vi) of the method the complex of the previous steps (comprising the exosome bound to a second interactor) is identified by performing a chemiluminescent reaction, wherein the identification results in a chemiluminescent emission of photons. Said chemiluminescent reaction may, for instance, be initiated by the addition of a trigger solution comprising, for example, H₂O₂ and NaOH. This results in an emission of photons, e.g., of a different wavelength according to the AE type used, which can be detected by a high sensitivity optical detector, preferably high sensitivity optical detector being part of the device according to the present invention.

In the context of the device of the present invention, said initiation of the chemiluminescence reaction may preferably occur in a cuvette as described herein.

In a last step (vii) the emission of photons is detected. This detection may be performed with any suitable chemiluminescent detector, for example a single-point detector such as a photomultiplier tube (PMT) an avalanche photodiode (APD), or a pixelated detector such as a charge-coupled device (CCD) or a complementary metal-oxide-semiconductor (CMOS) sensor.

The present invention envisages several variants and specific embodiments based on the above outlined general principles. For example, steps (ii) and (iii) as outlined above may be performed in different orders. In one embodiment, step (ii) is performed in a sequential order before step (iii), as defined above.

In an alternative embodiment, a reverse order is used, i.e. step (iii) is performed before step (ii). The corresponding variant of the method accordingly comprises the following order of steps:
- providing the exosome in an aqueous solution;
- interacting the exosome with at least one second interactor molecule linked to a chemiluminescent dye, wherein the interaction yields a complex comprising both the exosome and the second interactor molecule;
- interacting the complex of the previous step with at least one first interactor molecule;
- immobilizing the complex comprising the first and second interactor, e.g. via magnetic beads or on a substrate;
- performing a washing step;
- identifying the complex comprising the first and second interactor by performing a chemiluminescent reaction, wherein the identification results in a chemiluminescent emission of photons; and
- detecting the emission of photons.

Also envisaged is a simultaneous performance of steps (ii) and (iii). In these embodiments, first and second interactor molecules are interacted with the exosome at the same time.

Further envisaged are additional steps, which may be performed optionally. Such steps may include, for example one or more washing steps and/or one or more incubation step(s).

In certain embodiments of the invention the method is performed as multiplex detection. The multiplex approach is preferably based on the use of at least two, preferably at least three acridinium ester chemiluminescence dyes emitting light of a different wavelength, which can be linked to different first or second interactors and thus provide differential optical signals according to the exosome or exosome type identified. The different AE dyes are preferably present within one reaction zone, e.g. within one vessel or cuvette as described herein. The dyes are preferably selected from NSP-DMAE, HQYAE, TSPAE, NSP-LEZAE, Rhodamine-2-AM-DMAE or CNF-2-AM-DMAE, which emit radiation at different wavelengths as described herein.

In a corresponding particularly preferred embodiment of the multiplex approach of the invention the exosome comprises two or more, e.g. 2, 3 or 4 different surface markers, e.g. tetraspanins, the second interactor is two or more, e.g. 2, 3 or 4 different acridinium ester-labeled binding antibodies which binds to said two or more, e.g. 2, 3 or 4 different surface markers, e.g. tetraspanins. The first interactor molecule may be one or more binding proteins, e.g. antibodies, for one or more different surface markers, e.g. tetraspanins, at the surface of the exosome.

In the context of this embodiment, it is preferred that for each exosome type of interest a corresponding first interactor molecule and a corresponding second interactor molecule coupled to a different acridinium ester is used. This allows to distinguish the exosomes according to the different wavelengths of the acridinium esters such as NSP-DMAE, HQYAE, TSPAE, NSP-LEZAE, Rhodamine-2-AM-DMAE or CNF-2-AM-DMAE.

According to further specific embodiments of the present invention, the detection according to the present invention may be a qualitative detection, a semi-quantitative detection or a quantitative detection.

The term "qualitative detection" as used herein means that the method of the present invention is capable of indicating whether a specific exosome or exosome type is present or not. Accordingly, by detecting a signal via the bound antibody, it can be deduced that the exosome or exosome type is present, e.g. in a mixture of exosomes. A corresponding qualitative assay means that the assay can be used to achieve a yes or no answer indicating whether an exosome or exosome type is present in a sample. This may be achieved via a comparison versus a sample containing no exosomes or an unrelated control exosome.

The term "semi-quantitative detection" as used herein means that the method of the present invention is capable of indicating whether any exosome or a specific exosome or exosome type is present above a certain threshold with respect to its numbers or amount or concentration in a solution. The threshold may be suitably defined as would be known to the skilled person. For example, by detecting a signal in an assay on the basis of interactor molecules as described herein, it can de deduced that the exosomes or specific exosomes which are present, e.g. in a sample or a mixture of different exosomes has surpassed a certain predefined limit with respect to its numbers, amount or concentration. The format also allows to compare relative levels of exosomes between samples, in particular between samples used within the same assay format, or assays performed on platforms like Centaur or Atellica as mentioned herein above.

The term "quantitative detection" as used herein means that the method of the present invention is capable of indicating the approximate or exact numbers, amount or concentration of exosome or specific exosomes or exosome types. For the quantitative detection suitable control and/or calibration steps are required. Typically, the quantitative detection involves the interpretation of signals in comparison to a standard curve (e.g. a serial dilution of a known, purified exosomes) in order to precisely calculate the concentrations of exosomes in various samples. For example, in an assay, the signal data obtained may be graphed with chemiluminescent signal/light intensity vs. concentration to produce a standard curve. This data may subsequently be used to measure the concentration of unknown samples by comparison to the linear portion of the standard curve. This can advantageously be done directly on the graph or with suitable curve fitting software known to the skilled person. Based on positive calibrators a concertation curve of exosomes to detectable antibody signals, e.g. from acridinium esters, may be defined, which would allow for a suitable measurement of data.

It is further preferred that the quantitative detection is performed by averaging any replicates of the standards readings and by deducting the reading of the blank control sample. Subsequently, a standard curve is plotted and the line of best fit is identified so that the concentration of the samples can be determined. Any dilutions made need to be adjusted for at this stage. Alternatively, the signal data may be plotted using semi-log, log/log, log/logit or derivatives thereof in 4 or 5 parameter logistic models. Using software based/automated solutions suitable graphing approaches may be implemented. The approach further envisages the use of linear regression, e.g. within a software package, which allows for additional control possibilities. Further details would be known to the skilled person or can be derived from suitable literature sources.

In further preferred embodiments the method is performed in accordance with and/or on immunoassay platforms such as Centaur or Atellica as commercialized by Siemens Healthineers. Further information on these platforms may be derived, for example, from https://www.siemens-healthineers.com/immunoassay/systems/advia-centaur-xpt (visited on December 12, 2024) or https://www.healthcare.siemens.com/integrated-chemistry/systems/atellica-solution-analyzers (visited December 12, 2024.

In a very specific embodiment, the method may be performed by carrying out the following steps: pipetting of 100 µl sample into cuvette
- adding 100 µl of chemiluminescent reagent
- 40 minutes of incubation at 37°C (which allows for catching exosomes in sample, labeling of exosome-specific tetraspanins with AE-conjugated CD81 antibody)
- adding 50 µl Solid Phase
- 10 minutes of incubation at 37°C (which allows the biotinylated catcher-antibody to bind to streptavidin-coated magnetic beads)
- attracting magnetic beads with bound exosomes to cuvette wall via magnet
- wash with standard Atellica IM Wash Solution
- releasing the magnetic beads into cuvette
- adding 300 µl Atellica IM Acid Solution
- adding 300 µl Atellica IM Base Solution and immediately detect the emitted light flash via photomultiplier over 4 seconds.

Parameters as indicated above are chosen as illustration of one possible procedure or embodiment. The parameters can, in further embodiments of the invention, be changed or adapted.

In a further aspect the present invention relates to a kit for detecting exosomes in a sample comprising (1) a calibrator solution comprising a predefined number of exosomes; (2) a first interactor molecule, which is capable of interacting with an antigen at the surface of an exosome and the first interactor molecule; (3) a second interactor molecule, which is capable of interacting with an antigen at the surface of an exosome and which is linked to a chemiluminescent dye; (4) a magnetic bead comprising a third interactor molecule which is capable of interacting with the first interactor molecule by a biotin-streptavidin binding, wherein the first and second interactor molecule is an antibody against an exosome surface marker, preferably against tetraspanin.

The features of the methods as defined herein above apply also to the kit of the present invention. The kit may, for example, comprise reagents and components as defined in one or more steps of the present methods. The kit may, in general, comprise suitable buffer solutions or washing liquids etc. The kit may also comprise suitable substrates for detection. Furthermore, the kit may comprise an amount of a known exosome or exosome types, which can be used for a calibration of the kit or as an internal control. Corresponding ingredients would be known to the skilled person.

It is preferred that the first, second and third interactor molecule of the kit is the first, second and third interactor molecule as defined herein above in the context of the method of the present invention. It is further preferred that the first interactor molecule is an anti-tetraspanin antibody being covalently linked to biotin. It is further preferred that the second interactor molecule is an anti-tetraspanin antibody being linked to a chemiluminescent dye, preferably an acridinium ester. It is particularly preferred that the first or second interactor molecule an anti-CD81 antibody, an anti-CD63 antibody or an anti-CD9 antibody.

Additionally, the kit may comprise an instruction leaflet and/or may provide information as to its usage etc.

Also envisaged is an apparatus performing the above-mentioned method steps. The apparatus may, for example, be composed of different modules which can perform one or more steps of the method of the present invention. These modules may be combined in any suitable fashion, e.g., they may be present in a single place or be separated. Also envisaged is the performance of the method at different points in time and/or in different locations. Some steps of the method as defined herein may be followed by breaks or pauses, wherein the reagents or products etc. are suitably stored, e.g., in a freezer or a cooling device. In case these steps are performed in specific modules of an apparatus as defined herein, said modules may be used as storage vehicle. The modules may further be used to transport reaction products or reagents to a different location, e.g., a different laboratory etc.

The present invention further relates to the use of one or more first and second interactor molecules as defined herein above for the detection of exosomes in general, or exosome types or specific exosomes. The use is preferably for a detection assay.

The following examples and figures are provided for illustrative purposes. It is thus understood that the examples and figures are not to be construed as limiting. The skilled person in the art will clearly be able to envisage further modifications of the principles laid out herein.

### EXAMPLES

### EXAMPLE 1

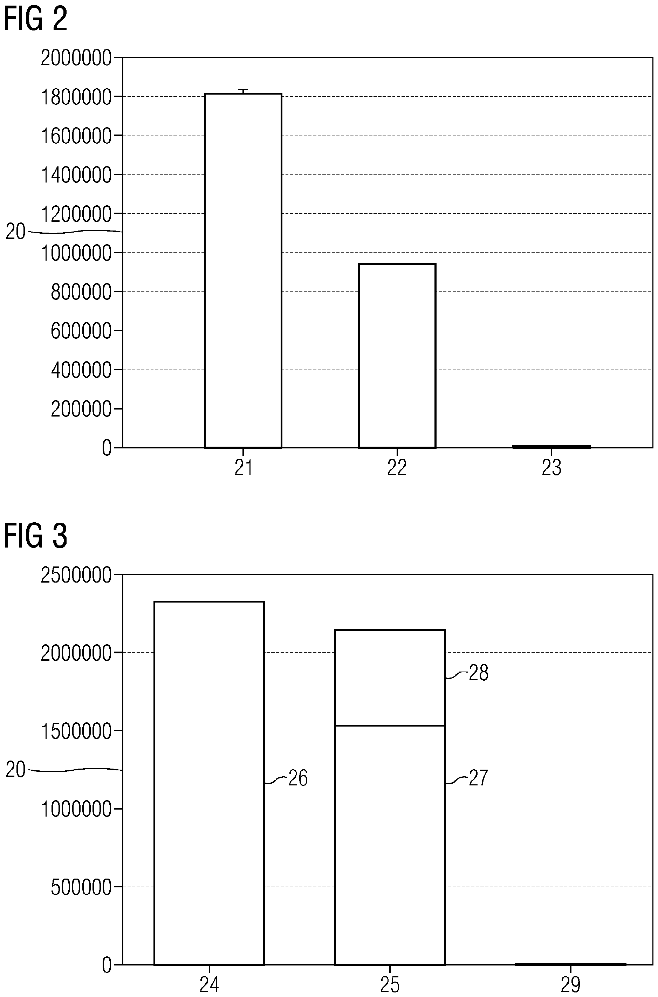

Assay specificity: An assay was performed wherein the assay setup showed high specificity, with positive signals in samples containing exosomes (e.g. human blood plasma ("plasma") or commercially available exosome standard, "ExoStd"), and very low background signals in pure buffer (1% BSA in PBS) as can be derived from Figure 2.

Exosome precipitation: an experiment was performed to examine the recovery of exosomes upon precipitation (see also Figure 3). The results indicate that there is only a very low background signal (1% BSA in PBS), while approximately 65% of the exosome signal measured in the input sample (plasma) is recovered in the precipitate. Besides that, also a positive signal could be detected in the "exofree" supernatant (26%), leading to a total recovery of 91% after precipitation.

### EXAMPLE 2

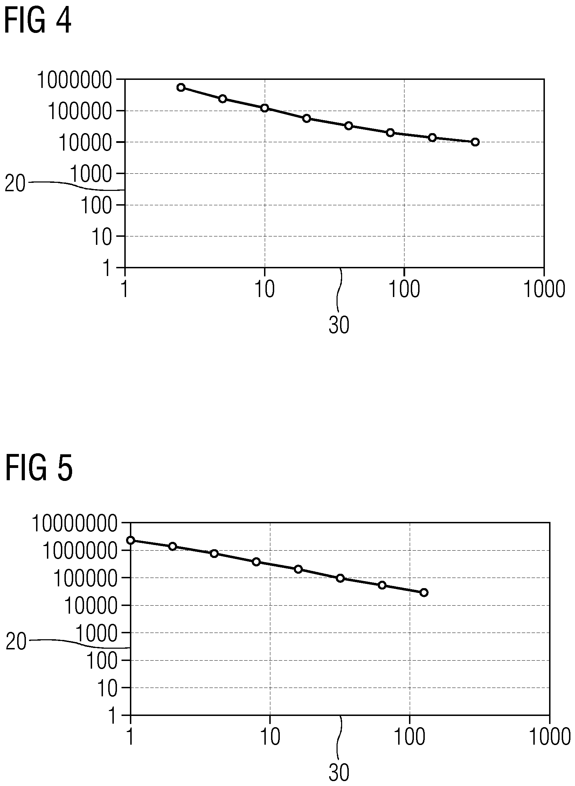

Sensitivity/Linearity of sample dilution: Linearity of serially diluted samples (either exosome standard (see Figure 4) or plasma in PBS (see Figure 5)) was assessed and found to be acceptable, while the coefficient of determination (R²) was detected to be higher in the method according to the invention when compared to commercially available ELISA tests (assumingly due to low sensitivity of ELISA).

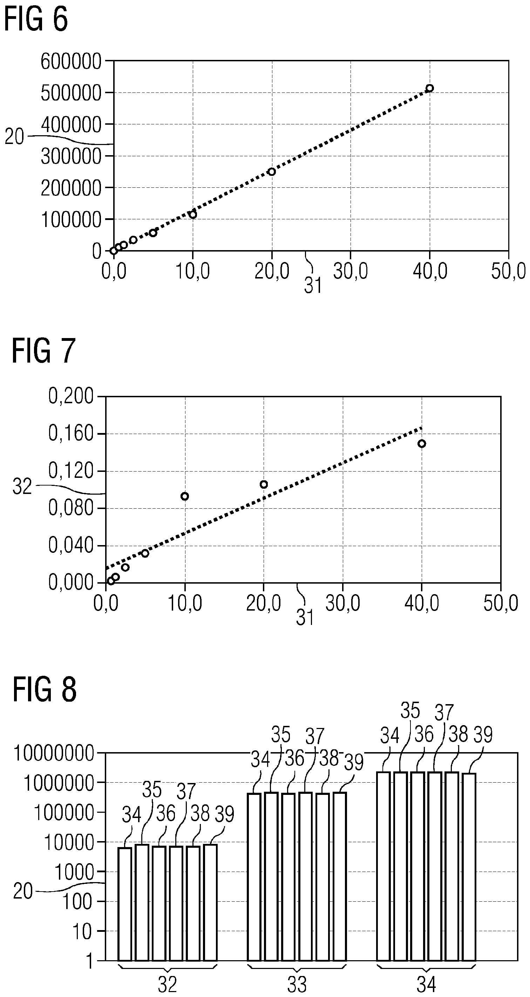

Serial dilution: The same ExoStd serial dilution was further measured with Atellica IM assay (see Figure 6) and ELISA (see Figure 7). It was found that the Atellica assay is significantly more sensitive and has a significantly higher linearity (R2) than ELISA.

In Figure 6 the displayed mass on the Atellica measurement is calculated from the original ExoStd concentration, based on NTA determination, as provided by the manufacturer.

### EXAMPLE 3

Assay precision / intra-assay variation: Intra-assay variation was determined by testing aliquots of 1% BSA in PBS (background; low signal), P1 IZON exosome isolation (intermediate signal), and P1 plasma (high signal). Six replicates of each sample were tested within the same run (measurements 1-6). As shown in Figure 8, all six measurements showed comparable RLUs, with coefficients of variation (CV%) of 14.2%, 4.3% and 1.8% respectively (see Table 1).

**Table 1:**

| **sample** | **mean** | **SD** | **CV%** |
|---|---|---|---|
| 1% BSA in PBS | 7531 | 1071 | 14.2% |
| P1 IZON | 448773 | 19278 | 4.3% |
| P1 Plasma | 2292367 | 41666 | 1.8% |

### EXAMPLE 4

Assay interference: Exosomes can be isolated by several methods, one of them being size exclusion chromatography. In the context of the present invention, IZON columns were used to separate exosomes from other plasma contents such as proteins and cell fragments.

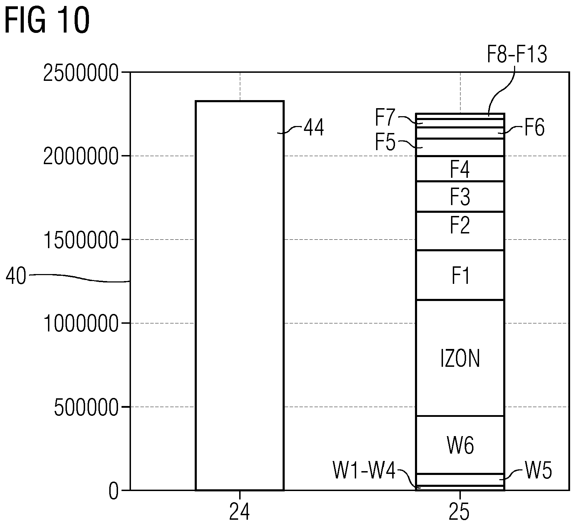

During the experiment the fraction with isolated exosomes ("IZON fraction") and also the fraction before (wash fractions W1-W6) and after (fractions F1-F13) were collected in order to obtain a full spectrum of the plasma contents. When collecting fractions accordingly, the wash fractions W1-W6 showed increasing amounts of exosome signal with increasing fraction number, while the "IZON" fraction (here the highest number of exosomes is suspected) has the highest signal (see Figures 9 and 10). Following post-IZON fractions F1-F13 showed decreasing exosome signals with increasing fraction number. A total of 96.8% of the input signal was recovered when summarizing signals obtained in all fractions.

On the other side, the amount of the smaller sized plasma proteins started to increase dramatically at F2 and the following fractions (protein determination was performed by bicinchoninic acid assay (BCA) including readout with a standard commercially available plate reader).

The IZON company showed very similar spectrums for their isolations, while IZON uses NTA for determination of particle numbers.

These results, in addition with the low signal in the supernatant of precipitated plasma samples shown above, suggest low risk of interference with other biological substances in plasma than exosomes. In other words, plasma proteins are considered not to interfere with the measurements.

## Claims

1. A method for detecting exosomes in a sample comprising the steps:
(i) providing the exosome in an aqueous solution;
(ii) interacting the exosome with at least one first interactor molecule, wherein the interaction yields a complex comprising both the exosome and the first interactor molecule;
(iii) interacting the complex of step (ii) with at least a second interactor molecule linked to a chemiluminescent dye;
(iv) interacting the complex of step (ii) or (iii) with a substrate or a bead and thereby immobilizing it;
(v) performing a washing step;
(vi) identifying the complex of step (iv) by performing a chemiluminescent reaction, wherein the identification results in a chemiluminescent emission of photons; and
(vii) detecting the emission of photons.

2. The method of claim 1, wherein the immobilization of the complex of step (ii) or (iii) is performed with a magnetic bead via a third interactor molecule which is present at the substrate or bead; or via the first interactor which is immobilized at the substrate ab initio.

3. The method of claim 2, wherein the first interactor molecule comprises biotin and the third interactor molecule comprises streptavidin and wherein the interaction is a biotin-streptavidin binding.

4. The method of claim 2 or 3, wherein the immobilization is performed via a magnetic force.

5. The method of any one of claims 1 to 4, wherein steps (ii) and (iii) are performed simultaneously, sequentially ((ii) before (iii)) or in reverse order ((iii) before (ii)), optionally followed and/or interrupted by one or more washing and/or incubation step(s).

6. The method of any one of claims 1 to 5, wherein the first and/or second interactor molecule is a binding protein, preferably an antibody.

7. The method of claim 6, wherein the antibody is an antibody against a specific antigen present at the surface of an exosome, such as a tetraspanin.

8. The method of claim 6 or 7, wherein the first and/or second interactor molecules are binding proteins or antibodies against at least two different antigens present at the surface of an exosome, preferably two or more different tetraspanins.

9. The method of claim 7 or 8, wherein the antibody is an anti-CD81 antibody, an anti-CD63 antibody or an anti-CD9 antibody.

10. The method of any one of claims 1 to 9, wherein the chemiluminescent dye is an acridinium ester, preferably NSP-DMAE, HQYAE, TSPAE, NSP-LEZAE, Rhodamine-2-AM-DMAE or CNF-2-AM-DMAE.

11. The method of claim 10, wherein the acridinium ester is covalently linked to the binding protein.

12. The method of claim 11, wherein the binding protein is an antibody.

13. The method of any one of claims 1 to 12, wherein said detection is a quantitative detection or a semi-quantitative detection.

14. The method of any one of claims 1 to 13, wherein the sample is a plasma, serum, urine, saliva, cell culture media or a fluid, preferably biofluid, which comprises exosomes.

15. A kit for detecting exosomes in a sample comprising (1) a calibrator solution comprising a predefined number of exosomes; (2) a first interactor molecule, which is capable of interacting with an antigen at the surface of an exosome and the first interactor molecule; (3) a second interactor molecule, which is capable of interacting with an antigen at the surface of an exosome and which is linked to a chemiluminescent dye; (4) a magnetic bead comprising a third interactor molecule which is capable of interacting with the first interactor molecule by a biotin-streptavidin binding, wherein the first and second interactor molecule is an antibody against an exosome surface marker, preferably against tetraspanin.
